# EUROPÄISCHE PATENTSCHRIFT

(11) **EP 0 540 556 B1**
(45) Veröffentlichungstag und Bekanntmachung des Hinweises auf die Patenterteilung: **30.11.1994**
(21) Anmeldenummer: 91912793.6
(22) Anmeldetag: 13.07.1991
(51) Int. Cl.: C12N 15/01, C12P 13/08

(54) **VERFAHREN ZUR HERSTELLUNG VON L-LYSIN PRODUZIERENDEN MIKROORGANISMEN**
METHOD OF PREPARATION OF L-LYSINE-PRODUCING MICROORGANISMS
PROCEDE POUR LA FABRICATION DE MICRO-ORGANISMES PRODUISANT DE LA L-LYSINE

(30) Priorität: 25.07.1990 DE 4023576
(43) Veröffentlichungstag der Anmeldung: 12.05.1993
(73) Patentinhaber: BASF Aktiengesellschaft, 67063 Ludwigshafen (DE)
(72) Erfinder: LADNER, Wolfgang, D-6701 Fussgoenheim (DE); PRESSLER, Uwe, D-6701 Altrip (DE); SIEGEL, Wolfgang, D-6800 Mannheim 1 (DE)
(86) Internationale Anmeldenummer: EP9101316
(87) Internationale Veröffentlichungsnummer: WO9201785

(56) Entgegenhaltungen:
- EP-A- 0 175 309
- Chemical Abstracts, vol. 89, no. 21, November 1978 (Columbus, Ohio, US), p. 456, abstract 178091f
- Chemical Abstracts, vol. 85, no. 13, 27 September 1976 (Columbus, Ohio, US), p. 469, abstract 92192v

## Beschreibung

Die vorliegende Erfindung betrifft ein Verfahren zur Herstellung von L-Lysin und Mikroorganismen dafür.

L-Lysin ist eine essentielle Aminosäure und findet als Zusatz zu Nahrungsmitteln und Tierfutter eine breite Anwendung. Es wird auch in der Medizin als Bestandteil von Infusionslösungen eingesetzt.

L-Lysin erhält man durch Hydrolyse von Proteinen mit Säure, durch Synthese von D,L-Lysin und anschließender Trennung des Racemats sowie durch Synthese mit Hilfe von Mikroorganismen. Mikrobiologische Verfahren zur Herstellung von L-Lysin sind beispielsweise beschrieben in Trends in Biotechnology 1 (1983), 70-74.

Es wurde nun ein verbessertes Verfahren zur Herstellung von L-Lysin produzierenden Mikroorganismen gefunden.

Gegenstand der Erfindung ist ein Verfahren zur Herstellung von Mikroorganismen, die eine erhöhte Produktivität für L-Lysin besitzen, durch Mutation von Mikroorganismen der Gattungen Corynebacterium und Brevibacterium mit bekannten Mutagenen in an sich bekannter Weise, dadurch gekennzeichnet, daß man Stämme der oben genannten Gattungen mutiert und solche selektioniert, die resistent gegen eine Rückkopplungshemmung durch 2-Azido-ε-caprolactam sind.

Überraschenderweise besitzt 2-Azido-ε-caprolactam eine wesentlich höhere Effizienz bei der Mutantenselektion nach Mutagenese als die in JP 51-19.186 und EP 175.309 beschriebenen Verbindungen, wie z.B. Fluor- oder Chlorcaprolactam.

So läßt sich durch Selektion mit Azidocaprolactam die Lysinproduktivität von Stämmen um mehr als 10 % steigern.

Die erfindungsgemäßen Mutanten lassen sich durch konventionelle Mutagenese, z.B. mit N-Methyl-N'-nitro-N-nitrosoguanidin oder durch U.V.-Bestrahlung herstellen.

Als Mikroorganismen der Gattungen Corynebacterium (C) und Brevibacterium (B) eignen sich beispielsweise:
B. amoniagenis, B. divaricatum, B. flavum, B. ketoglutamicum, B. lactofermentum, B. linens, B. sp., C. acetoacidophilum, C. acetoglutamicum, C. glutamicum, C. lilium und C. sp. Bevorzugt sind B. flavum und C. glutamicum, insbesondere B. flavum ATCC 21.474 und C. glutamicum ATCC 21.526. Letzterer hat den besonderen Vorteil, daß er Homoserinbedürftig ist und außerdem resistent gegen S-(2-Aminoethy)-L-cystein ist.

Wie bereits angedeutet, ist es günstig, wenn die Stämme Homoserin-bedürftig sind. Weiter ist es gut, wenn die Stämme auch resistent gegen S-(2-Aminoethyl)L-cystein sind. Falls diese Resistenz nicht in den Stämmen vorhanden ist, läßt sie sich gemäß US 3,707,441 durch Behandlung der Stämme mit N-Methyl-N'-nitro-N-nitrosoguanidin und anschließende Selektion einführen.

### Beispiel

Corynebacterium glutamicum ATCC 21 526 wurde in Tris/Maleinsäure-Puffer, pH 6,0, 30 min bei 30°C mit 250 µg/ml N-Methyl-N'-nitro-N-nitrosoguanidin behandelt. Anschließend wurden die Zellen mit 0,1 M Trispuffer pH 7,2 gewaschen, auf Minimalagar-Platten plattiert und danach 4 bis 14 Tage bei 28°C inkubiert.

Der Minimalagar hatte folgende Zusammensetzung:

| | |
|---|---|
| 20 g/l Agar | 0,1 g/l MnS0₄·H₂O |
| 2 g/l (NH₄)₂SO₄ | 100 µg/l Biotin |
| 0,5 g/l KH₂PO₄ | je 30 mg/l Met, Thr, Leu |
| 0,5 g/l K₂HPO₄ | 4 g/l Lactat |
| 0,4 g/l MgSO₄·7H₂O | |
| 0,01 g/l FeSO₄·7H₂O | pH = 7,0 |

Aus den Kolonien, die nach Inkubation auf den Agarplatten wuchsen, wurden diejenigen ermittelt, die Lysin produzierten. Die Stämme, die 10 % mehr Lysin als der Ausgangsstamm bildeten, wurden isoliert.

## Patentansprüche

1. Verfahren zur Herstellung von Mikroorganismen, die eine erhöhte Produktivität für L-Lysin besitzen, durch Mutation von Mikroorganismen der Gattungen Corynebacterium und Brevibacterium mit bekannten Mutagenen in an sich bekannter Weise, dadurch gekennzeichnet, daß man Stämme der oben genannten Gattungen mutiert und solche selektioniert, die resistent gegen eine Rückkopplungshemmung durch 2-Azido-ε-caprolactam sind.

## Claims

1. A process for producing microorganisms which have increased L-lysine productivity by mutation of microorganisms of the genera Corynebacterium and Brevibacterium with known mutagens in a conventional manner, which comprises mutating strains of the abovementioned genera and selecting those resistant to feedback inhibition by 2-azido-ε-caprolactam.

## Revendications

1. Procédé de préparation de microorganismes qui possèdent une productivité élevée pour L-lysine, par mutation de microorganismes des genres Corynebacerium et Brevibacterium avec des agents mutagènes connus, de manière connue en soi, caractérisé par le fait qu'on mute les souches des genres sus-indiqués et on sélectionne celles qui sont résistantes à une inhibition de contre-réaction par 2-azido-ε-caprolactame.
